# EUROPEAN PATENT SPECIFICATION

(11) **EP 1 627 639 B1**
(45) Date of publication and mention of the grant of the patent: **23.12.2009**
(21) Application number: 05024864.0
(22) Date of filing: 31.05.2002
(51) Int. Cl.: A61K 31/635, A61K 31/365, A61K 31/54, A61K 31/435, A61K 31/42, A61P 25/24, A61K 45/06

(54) **Use of COX-2 inhibitors for the treatment of affective disorders**
Verwendung von COX-2 Inhibitoren zur Behandlung von affectiven Störungen
Utilisation de COX-2 inhibiteurs pour traiter les troubles affectives

(30) Priority: 19.06.2001 DE 10129320; 14.03.2002 US 364904 P
(43) Date of publication of application: 22.02.2006
(62) Divisional of application: 02738138.3
(73) Proprietor: Müller, Norbert, 80686 München (DE)
(72) Inventor: Müller, Norbert, 80686 München (DE)
(74) Representative: Wibbelmann, Jobst

(56) References cited:
- EP-A- 1 142 889
- WO-A-99/25353
- "Adverse effects of rofecoxib (continued)" PRESCRIRE INTERNATIONAL 2001 FRANCE, vol. 10, no. 53, 2001, pages 82-83, XP008067896 ISSN: 1167-7422
- O'CONNOR J J ET AL: "NS-398, the cyclooxygense-2 inhibitor attenuates long term depression in the rat dentate gyrus in vitro." SOCIETY FOR NEUROSCIENCE ABSTRACTS, vol. 27, no. 1, 2001, page 1025, XP001109452 31st Annual Meeting of the Society for Neuroscience;San Diego, California, USA; November 10-15, 2001 ISSN: 0190-5295
- LANTZ M S (REPRINT) ET AL: "Acute onset of auditory hallucinations after initiation of celecoxib therapy" AMERICAN JOURNAL OF PSYCHIATRY, (JUN 2000) VOL. 157, NO. 6, PP. 1022-1023 PUBLISHER: AMER PSYCHIATRIC PRESS, INC, 1400 K ST, N W, STE 1101, WASHINGTON, DC 20005. ISSN: 0002-953X., 2000, XP001133838
- JIANG H-K ET AL: "NON-STEROIDAL ANTI-INFLAMMATORY DRUGS WITH ADVERSE PSYCHIATRIC REACTIONS: FIVE CASE REPORTS" CLINICAL RHEUMATOLOGY, ACTA MEDICA BELGICA, BRUXELLES, BE, vol. 18, no. 4, 1999, pages 339-345, XP001007036 ISSN: 0770-3198
- SRAMEK, JOHN J. ET AL: "The status of ongoing trials for mild cognitive impairment" EXPERT OPINION ON INVESTIGATIONAL DRUGS (2001), 10(4), 741-752, 2001, XP008012341
- COLLANTES-ESTEVEZ: "Improved control..." CURRENT MEDICAL RESEARCH AND OPINION, vol. 19, no. 5, 2003, pages 402-410,
- JOHNSTON: BRIT J. PSYCHIATR, 139:89-101, - 1981
- LINDENMAYER: PSYCHIATR. QUART. 65:299-322, 1994,
- KAY: PSYCHIATR. QUART. 61:163-178, 1990,
- KIM ET AL.: PSYCHIATR. RES. 144: 57-63, 2006,
- TRUMMLITZ: MILESTONES IN DRUG THERAPY, 2004,

## Description

This is a divisional application of EP 1 397 145 (application no. 02 738138.3)

The invention concerns the use of a COX-2 (cyclooxygenase-2) inhibitor according to the claims for the treatment of depression, depressive episodes, recurring depressive episodes, manic episodes and bipolar affective disorders.

Moreover, the invention is concerned with the use of a COX-2 inhibitor according to the claims in combination with a neuroleptic drug or an antidepressant for the treatment of these psychiatric disorders.

A relation between immunological dysfunctions and psychotic diseases, such as schizophrenia or affective disorders, has been discussed controversially over the last century.

In the case of schizophrenia for instance the pathogenesis is still unknown, but many findings indicate that schizophrenia is a syndrome based on different pathogenetic processes.

An inflammatory/immunological pathogenesis has been discussed for a subgroup of schizophrenic patients (Yolken RH, Torrey EF: Viruses, schizophrenia, and bipolar disorder. Clin Microbiol Rev 1995; 8:131-145; Körschenhausen D, Hampel H, Ackenheil M, Penning R, Müller N: Fibrin degradation products in post mortem brain tissue of schizophrenics: a possible marker for underlying inflammatory processes, Schizophr Res 1996; 19: 103-109; Müller N, Ackenheil M: Psychoneuroimmunology and the cytokine-network in the CNS: implications for psychiatric disorders. Prog Neuropsychopharmacol & Biol Psychiat 1998; 22: 1-33). Studies showed that activating cytokines like interleukin-1 (IL-1) and IL-2 are increased in the cerebrospinal fluid of schizophrenic patients compared to controls (Sirota P, Schild K, Elizur A, Djaldetti M, Fishman P: Increased Interleukin-1 and Interieukin-3 like activity in schizophrenic patients. Prog Neuropsychopharmacol & Biol Psychiatry 1995; 19: 85-83; Licinio J, Seibyl, JP, Altemus M, Chamey DS, Krystal JH: Elevated levels of Interleukin-2 in neuroleptic-free schizophrenics. Am J Psychiatry 1993; 150: 1408-1410), and that high levels of IL-2 in the cerebrospinal fluid are a predictor for the increased probability of a schizophrenic relapse (McAllister CG, van Kamen DP, Rehn TJ, Miller AL, Gurklis J, Kelley ME, Yao J, Peters JL: Increases in CSF levels of Interleukin-2 in schizophrenia: effects of recurrence of psychosis and medication status. Am J Psychiatry 1995; 152: 1291-1297).

On the other hand, in a subgroup of schizophrenic patients a decreased immune response compared to controls has been observed, possibly due to a disturbance of antigen-presentation or antigen-recognition (Schwarz MJ, Riedel M, Ackenheil M, Müller N: Decreased levels of soluble intercellular adhesion molecule -1 (sICAM-1) in unmedicated and medicated schizophrenic patients. Biol Psychiatry 2000; 47: 29-33), e.g. the increased immune reaction in the central nervous system may not be adequately regulated by an immune reaction in the peripheral immune system. This was observed mostly in acute schizophrenic patients presenting a recent onset of the disorder.

Another group of schizophrenic patients, however, seems to present an over-activation of the peripheral immune system in the sense of autoimmune processes (Radaport MH, Müller N: Immunological states associated with schizophrenia. In: Ader R, Felten DL, Cohen N (eds) Psychoneuroimmunology, Third Edition. Vol. 2, San Diego, Academic Press, 2001; pp 373-382; Radaport MH, McAllister CG, Kim YS, Han JH, Pickar D, Nelson DM, Kirch DG, Paul SM: Increased soluble Interleukin-2 receptors in Caucasian and korean schizophrenic patients. Biol Psychiatry 1994; 35: 767-771). In several studies, increased titers of antibodies against the heat-shock-protein 60 were observed (Kilidireas K, Latov N, Strauss DH, Aviva DG, Hashim GA, German JM, Sadiq SA: Antibodies to human 60 KD hear-shock protein in patients with schizophrenia. Lancet 1992; 340: 569-572), the increase being accompanied by increased soluble IL-2 receptors in the serum and increased titers of the soluble adhesion molecule sICAM-1 (Radaport MH, Müller N: Immunological states associated with schizophrenia. In: Ader R, Felten DL, Cohen N (eds) Psychoneuroimmunology, Third Edition. Vol. 2, San Diego, Academic Press, 2001; pp 373-382; Schwarz MJ, Riedel M, Gruber R, Ackenheil M, Müller N: Antibodies to heat-shock proteins in schizophrenic patients - Implications for disease mechanism. Am J Psychiatry 1999; 156, 1103,1104). The close relationship between high sVCAM-1 titers and more pronounced schizophrenic negative symptoms (Schwarz MJ, Riedel M, Gruber R, Ackenheil M, Müller N: Levels of soluble adhesion molecules in schizophrenia: Relation to psychopathology. In: N. Müller (Hrg) Psychiatry, Psychoneuroimmunology, and Viruses. Springer Verlag Wien, 1999; NY, pp. 121-130) as well as between high IgG levels in the cerebrospinal fluid and more pronounced negative symptoms further support this observation (Müller N, Ackenheil M: Immunoglobulin and albumin contents of cerebrospinal fluid in schizophrenic patients: The relationship to negative sympomatology. Schizophrenia Res 1995; 14: 223-228).

Affective diseases, in particular depressive diseases, may also have an inflammatory genesis. This is manifested in the fact that general inflammatory diseases are accompanied by depressive syndromes to an increased extent as well as in the fact that in depressive diseases, signs of inflammation occur more frequently in comparison to psychologically healthy persons. Scientifically, this was expressed in the monocyte/macrophage hypothesis of depression.

The occurrence of tics as well as of autism has also been discussed in many cases as a consequence of inflammatory processes.

The invention is based on the idea that substances with immunomodulatory properties could be used for the treatment of psychiatric disorders such as schizophrenia, delusional disorders, affective disorders, autism or tic disorders, which are at least partially based on immunological pathogenetic processes.

For example, in the treatment of schizophrenia, a number of neuroleptic drugs (so-called classical and atypical neuroleptics) have become available, among which the more recent atypical neuroleptics excel by comparatively good effectiveness with a more favorable side effect profile. Unlike the classical neuroleptics; which are mainly effective for treating the positive symptoms of schizophrenia, the atypical neuroleptics improve both positive symptoms (hallucinations, delusions, and conceptual disorganization) and negative symptoms (apathy, social withdrawal, affective flattening, and poverty of speech) of schizophrenia. Plus, presumably due to their altered receptor binding profile, the atypicals cause minimal extrapyramidal symptoms and rarely cause tardive dyskinesias.
Anyhow, neuroleptics in general act as syndrome oriented therapy and less as a causal therapy.

Therefore, a need exists for further medicaments for the treatment of affective disorders.

The present invention is directed to the use of COX-2 inhibitors as identified in the claims for the manufacture of a medicament for the treatment of depression, depressive episodes, recurring depressive episodes, manic episodes and bipolar affective disorders.
In the context of the present invention a treatment of a disease or disorder is meant to cover the actual therapy as well as maintenance therapy and prophylaxis against recurrence.

Furthermore, the invention concerns the use of the COX-2 inhibitors as identified in the claims in combination with neuroleptics or antidepressants for the treatment of the above affective disorders.

The invention is also directed to kit-of-parts that is suitable for use in the treatment of the disorders identified above, the kit comprising a first dosage form comprising a neuroleptic or an antidepressant and a second dosage form comprising a COX-2 inhibitor according to the claims for simultaneous, separate or sequential administration.

The COX-2 inhibitors of the present invention belong to the class of nonsteroidal antiinflammatory drugs (NSAIDs). It has been known for some time that many of the common NSAIDs modulate prostaglandin synthesis by inhibition of cyclooxygenases that catalyze the transformation of arachidonic acid - the first step in the prostaglandin synthesis pathway. However, the use of high doses of many common NSAIDs can produce severe side effects that limit their therapeutic potential. In an effort to reduce the unwanted side effects of common NSAIDS, it was discovered that two cyclooxygenases are involved in the transformation of arachidonic add as the first step in the prostaglandin synthesis pathway. These enzymes have been termed cyclooxygenase-1 (COX-1) and cyclooxygenase-2 (COX-2)(Needleman, P. et al., J. Rheumatol., 24, Suppl.49:6 - 8 (1997);Fu, J. Y., et al., J. Biol. Chem., 265(28):16737-40 (1990)). COX-1 has been shown to be a constitutively produced enzyme that is involved in many of the non-inflammatory regulatory functions associated with prostaglandins. COX-2, on the other hand, is an inducible enzyme having significant involvement in the inflammatory process. Inflammation causes the induction of COX-2, leading to the release of prostanoids, which sensitize peripheral nociceptor terminals and produce localized pain hypersensitivity (Samad, T. A. et al., Nature, 410(6827):471-5 (2001)). Many of the common NSAIDs are now known to be inhibitors of both COX-1 and COX-2. Accordingly, when administered in sufficiently high levels, these NSAIDs affect not only the inflammatory consequences of COX-2 activity, but also the beneficial activities of COX-1. Recently, compounds that selectively inhibit COX-2 to a greater extent than the activity of COX-1 have been discovered. These new COX-2 inhibitors are believed to offer advantages that include the capacity to prevent or reduce inflammation while avoiding harmful side effects associated with the inhibition of COX-1, such as gastrointestinal and renal side effects, as well as inhibition of thrombocyte aggregation.

The use of COX-2 inhibitors in the therapy of arthritis and related indications is known. US 5,760,068 describes the use of COX-2 inhibitors for the treatment of rheumatoid arthritis and osteoarthritis. WO 00/32189 discloses the preparation of pharmaceutical compositions containing the COX-2 inhibitor celecoxib and the use of celecoxib for the treatment of rheumatoid arthritis or as a painkiller.

The term COX-2 inhibitor embraces compounds which selectively inhibit cyclooxygenase-2 over cyclooxygenase-1, and also includes pharmaceutically acceptable salts thereof.

In accordance with the invention the cyclooxygenase inhibitor is selected from the class of tricyclic COX-2 inhibitors represented by the general structure of Formula: 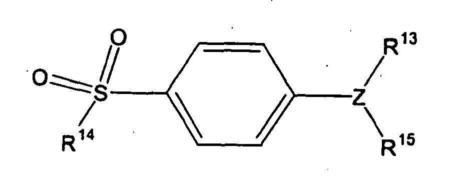 wherein:
Z is selected from the group consisting of partially unsaturated or unsaturated heterocyclyl and partially unsaturated or unsaturated carbocyclic rings;
R¹³ is selected from the group consisting of heterocyclyl, cycloalkyl, cycloalkenyl and aryl, wherein R¹³ is optionally substituted at a substitutable position with one or more radicals selected from alkyl, haloalkyl, cyano, carboxyl, alkoxycarbonyl, hydroxyl, hydroxyalkyl, haloalkoxy, amino, alkylamino, arylamino, nitro, alkoxyalkyl, alkylsulfinyl, halo, alkoxy and alkylthio;
R¹⁴ is selected from the group consisting of methyl or amino; and
R¹⁵ is selected from the group consisting of a radical selected from H, halo, alkyl, alkenyl, alkynyl, oxo, cyano, carboxyl, cyanoalkyl, heterocyclyloxy, alkyloxy, alkylthio, alkylcarbonyl, cycloalkyl, aryl, haloalkyl, heterocyclyl, cycloalkenyl, aralkyl, heterocyclylalkyl, acyl, alkylthioalkyl, hydroxyalkyl, alkoxycarbonyl, arylcarbonyl, aralkylcarbonyl, aralkenyl, alkoxyalkyl, arylthioalkyl, aryloxyalkyl, aralkylthioalkyl, aralkoxyalkyl, alkoxyaralkoxyalkyl, alkoxycarbonylalkyl, aminocarbonyl, aminocarbonylalkyl, alkylaminocarbonyl, N- arylaminocarbonyl, N-alkyl-N-arylaminocarbonyl, alkylaminocarbonylalkyl, carboxyalkyl, alkylamino, N-arylamino, N-aralkylamino, N-alkyl-N-aralkylamino, N-alkyl-N-arylamino, aminoalkyl, alkylaminoalkyl, N-arylaminoalkyl, N-aralkylaminoalkyl, N-alkyl-N-aralkylaminoalkyl, N-alkyl-N-arylaminoalkyl, aryloxy, aralkoxy, arylthio, aralkylthio, alkylsulfinyl, alkylsulfonyl, aminosulfonyl, alkylaminosulfonyl, N-arylaminosulfonyl, arylsulfonyl, N-alkyl-N-arylaminosulfonyl.

In a preferred embodiment of the invention the COX-2 inhibitor represented by the above Formula is selected from the group of compounds, illustrated in Table 1, which includes celecoxib (B-18), valdecoxib (B-19), deracoxib (B-20), rofecoxib (B-21), etoricoxib (MK-663; B-22), JTE-522 (B-23).

Additional information about selected examples of the COX-2 inhibitors discussed above can be found as follows: celecoxib (CAS RN 169590-42-5, C-2779, SC-58653, and in U.S. Patent No. 5,466,823); deracoxib (CAS RN 169590-41-4); rofecoxib (CAS RN 162011-90-7); compound B-24 (U.S. Patent No. 5,840,924); compound B-26 (WO 00/25779); and etoricoxib (CAS RN 202409-33-4, MK-663, SC-86218, and in WO 98/03484).

**Table 1. Examples of Tricyclic COX-2 Inhibitors as Embodiments**

| **Compound Number** | **Structural Formula** |
|---|---|
| B-18 | 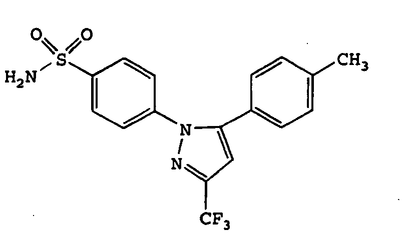 |
| B-19 | 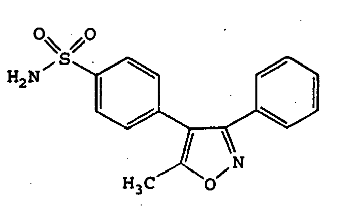 |
| B-20 | 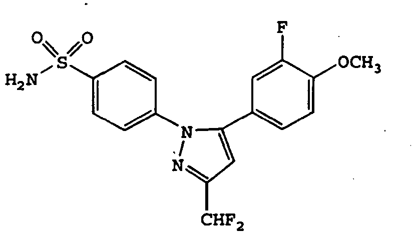 |
| B-21 | 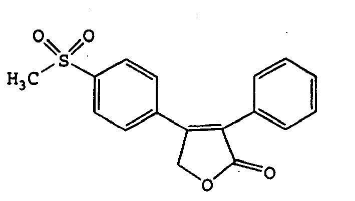 |
| B-22 | 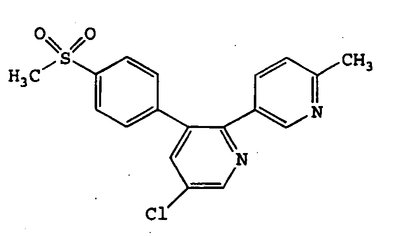 |
| B-23 | 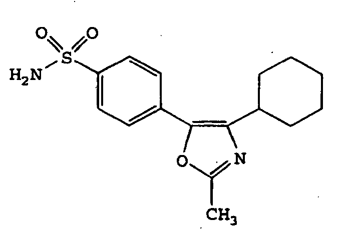 |

In a more preferred embodiment of the invention, the COX-2 inhibitor is selected from the group consisting of celecoxib, rofecoxib and etoricoxib. In a preferred embodiment of the invention, parecoxib (U.S. Patent No. 5,932,598), having the structure shown in B-24, which is a therapeutically effective prodrug of the tricyclic COX-2 inhibitor valdecoxib, B-19, (U.S. Patent No. 5,633,272), may be advantageously employed as a source of a cyclooxygenase inhibitor. A preferred form of parecoxib is sodium parecoxib.

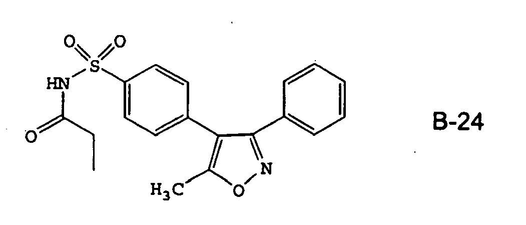

In another preferred embodiment of the invention, the compound ABT-963 having the formula B-25 that has been previously described in International Publication number WO 00/24719, is another tricyclic COX-2 inhibitor which may be advantageously employed.

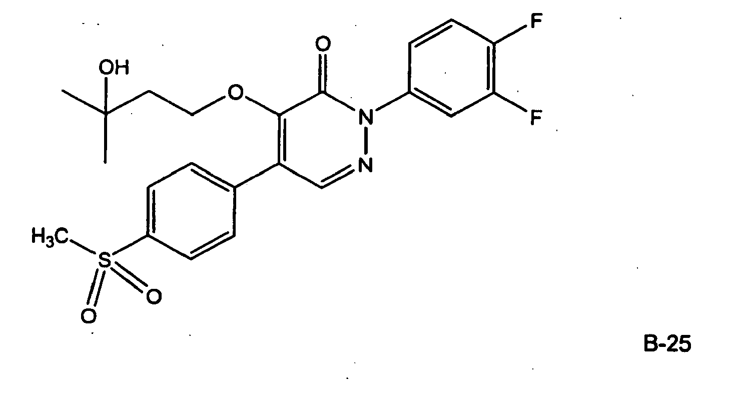

Preferred COX-2 inhibitors for the use according to the present invention include celecoxib (Celebrex®), rofecoxib (Vioxx®), deracoxib, parecoxib, valdecoxib, etoricoxib, ABT963, JTE-522, pharmaceutically acceptable salts or mixtures thereof. More preferred COX-2 inhibitors are celecoxib, parecoxib, valdecoxib, etoricoxib and rofecoxib.

According to a preferred embodiment, celecoxib (Celebrex®) or a pharmaceutically acceptable salt thereof is used. The term pharmaceutically acceptable salt includes salts that can be prepared according to known methods by those skilled in the art from the corresponding compound of the present invention, e.g. conventional metallic ion salts and organic salts.

Celecoxib can be administered at a dose of 50-1600 mg per day, preferably 200 to 600 mg, most preferably 400 mg per day. The administration can be carried out once or several times a day, preferably twice. The amount of celecoxib can be adapted depending on age, body weight and/or possible other diseases of the patient. Preferably, celecoxib is used in the form of tablets (Celebrex®) for oral administration.

Without intending to establish a certain theory as explanation for the observed effect of COX-2 inhibitors, the following mechanisms of action are taken into consideration.

There is no doubt that activation of COX-2 mediates inflammatory processes and that COX-2 is expressed in brain tissue. COX-2 can be activated by cytokines like IL-2, IL-6 and IL-10, and cytokine-activated COX-2 expression mediates further inflammatory processes. It was reported that IL-2 and soluble IL-2 receptors (Licino J, Seibyl, JP, Altemus M, Charney DS, Krystal JH: Elevated levels of Interleukin-2 in neuroleptic-free schizophrenics. Am J Psychiatry 1993; 150: 1408-1410) (McAllister CG, van Kemmen DP, Rehn TJ, Miller AL, Gurklis J, Kelley ME, Yao J, Peters JL: Increases in CSF levels of Interleukin-2 in schizophrenia: effects of recurrence of psychosis and medication status. Am J Psychiatry 1995; 152: 1291-1297), soluble IL-6 receptors as a functional part of the IL-6 system (Müller N, Dobmeier P, Empel M, Riedel M, Schwarz M, Ackenheil M: Soluble IL-6 Receptors in the serum and cerebrospinal fluid of paranoid schizophrenic patients. Eur Psychiatry 1997; 12: 294-299) and IL-10 (Van Kammen DP, McAllister-Sistilli CG, Kelley ME: Relationship between immune and behavioral measures in schizophrenia. In: G. Wieselmann (ed.) Current Update in Psychoimmunology, Springer Verlag 1997; Wien, NY, pp. 51-55) are increased in the cerebrospinal fluid of schizophrenic patients - the increase of the cytokines in the CNS may be accompanied by increased COX-2 expression. The effectiveness of COX-2 inhibitors, such as celecoxib, in the treatment of schizophrenia, might be based on the finding that celecoxib down-regulates the cytokine-induced CNS COX-2 activation.

Moreover, COX-2 inhibition seems to regulate the expression of adhesion molecules (Schwarz MJ, Ackenheil M, Riedel M, Müller N: Blood-CSF-Barrier impairment as indicator for an immune process in schizophrenia. Neurosci Letters 1998; 253: 201-203). Since adhesion molecule regulation is impaired in schizophrenia, leading to dysbalance and lack of communication between the peripheral and the CNS immune system, the effects of COX-2 inhibitors, such as celecoxib, in the treatment of schizophrenia, may also be related to the adhesion molecules ICAM-1 and VCAM-1, expecially regarding the negative symptoms (Schwarz MJ, Riedel M, Gruber R, Ackenheil M, Müller N: Levels of soluble adhesion molecules in schizophrenia: Relation to psychopathology. In: N. Müller (Hrg) Psychiatry, Psychoneuroimmunology, and Viruses. Springer Verlag Wien, 1999, NY, pp. 121-130; Müller N, Ackenheil M: Immunoglobulin and albumin contents of cerebrospinal fluid in schizophrenic patients: The relationship to negative sympomatology. Schizophrenia Res 1995; 14: 223-228).

According to a further embodiment of the present invention, a COX-2 inhibitor as defined above and in the claims is used in combination with a neuroleptic drug or an antidepressant for the manufacture of a medicament for the treatment of affective disorders. Combinations can also include a mixture of one or more COX-2 inhibitors with one or more neuroleptic agents or antidepressants. In particular, the combination of a COX-2 inhibitor with a neuroleptic drug is useful for the treatment of schizophrenia, whereas the combination of a COX-2 inhibitor with an antidepressant is applicable for the treatment of depressive disorders.

Both classical and atypical neuroleptics can be used for the add-on use according to the invention, atypical neuroleptics being preferred.

Examples of neuroleptic drugs that are useful in the present invention include: butyrophenones, such as haloperidol, pimozide, and droperidol; phenothiazines, such as chlorpromazine, thioridazine, mesoridazine, trifluoperazine, perphenazine, fluphenazine, thiflupromazine, prochlorperazine, and acetophenazine; thioxanthenes, such as thiothixene and chlorprothixene; thienobenzodiazepines; dibenzodiazepines; benzisoxazoles; dibenzothiazepines; imidazolidinones; benzisothiazolyl-piperazines; dibenzoxazepines, such as loxapine; dihydroindolones, such as molindone; aripiprazole; and derivatives thereof that have antipsychotic activity.
Examples of neuroleptic drugs that are preferred for use in the present invention are shown in Table 2.

**Table 2: Neuroleptic drugs**

| **Common Name** | **Trade Name** | **Route of Administration** | **Form** | **Dosage Range and (Median)^{a}** |
|---|---|---|---|---|
| Clozapine | CLOZARIL | oral | tablets | 12.5-900 mg/day |
| | | | | (300-900 mg/day) |
| Olanzapine | ZYPREXA | oral | tablets | 5-25 mg/day |
| | | | | (10-25 mg/day) |
| Ziprasidone | GEODON | oral | capsules | 20-80 mg/twice a day |
| | | | | (80-160 mg/day) |
| Risperidone | RISPERDAL | oral | solution tablets | 2-16 mg/day |
| | | | | (4-12 mg/day) |
| Quetiapine fumarate | SEROQUEL | oral | tablets | 50-900 mg/day |
| | | | | (300-900 mg/day) |
| Sertindole | SERLECT | | | (4-24 mg/day) |
| Amisulpride | | | | |
| Haloperidol | HALDOL | oral | tablets | 1-100 mg/day |
| | | | | (1-15 mg/day) |
| Haloperidol Decanoate | HALDOL Decanoate | parenteral | injection | |
| Haloperidol lactate | HALDOL INTENSOL | oral | solution | |
| | | parenteral | injection | |
| Chlorpromazine | THORAZINE | rectal | suppositori es | 30 - 800 mg/day |
| | | oral | capsules solution tablets | (200-500 mg/day) |
| | | parenteral | injection | |
| Fluphenazine | PROLIXIN | | | 0.5 - 40 mg/day (1-5 mg/day) |
| Fluphenazine decanoate | PROLIXIN Decanoate | parenteral | injection | (about one-half the dosage shown for oral) |
| Fluphenazine enanthate | PROLIXIN | parenteral | injection | (same as above) |
| Fluphenazine hydrochloride | PROLIXIN | oral | elixer solution tablets | |
| | | parenteral | injection | |
| Thiothixene | NAVANE | oral | capsules | 6-60 mg/day (8 - 30 mg/day) |
| Thiothixene hydrochloride | NAVANE | oral | solution | |
| | | parenteral | injection | |
| Trifluoperazine | STELAZINE | | | (2-40 mg/day) |
| Perphenazine | TRILAFON | oral | solution tablets | 12-64 mg/day |
| | | | | (16-64 mg/day) |
| | | parenteral | injection | |
| Perpehazine and Amitriptyline hydrochloride | ETRAFON TRIAVIL | oral | tablets | |
| Thioridazine | MELLARIL | oral | suspension solution tablets | 150-800 mg/day |
| | | | | (100 - 300 mg/day) |
| Mesoridazine | | | | (30 - 400 mg/day) |
| Molindone | MOBAN | | | 50-225 mg/day |
| | | | | (15-150 mg/day) |
| Molindone hydrochloride | MOBAN | oral | solution | |
| Loxapine | LOXITANE | | | 20 - 250 mg/day |
| | | | | (60-100 mg/day) |
| Loxapine hydrochloride | LOXITANE | oral | solution | |
| | | parenteral | injection | |
| Loxapine succinate | LOXITANE | oral | capsules | |
| Pimozide | | | | (1-10 mg/day) |
| Flupenthixol | | | | |
| Promazine | SPARINE | | | |
| Triflupromazine | VESPRIN | | | |
| Chlorprothixene | TARACTAN | | | |
| Droperidol | INAPSINE | | | |
| Acetophenazine | TINDAL | | | |
| Prochlorperazine | COMPAZINE | | | |
| Methotrimeprazine | NOZINAN | | | |
| Pipotiazine | PIPOTRIL | | | |
| Ziprasidone | | | | |
| Hoperidone | | | | |
| Zuclopenthixol | | | | |

Examples of tradenames and suppliers of selected neuroleptic drugs are as follows: clozapine (available under the tradename CLOZARIL®, from Mylan, Zenith Goldline, UDL, Novartis); olanzapine (available under the tradename ZYPREXA®, from Lilly; ziprasidone (available under the tradename GEODON®, from Pfizer); risperidone (available under the tradename RISPERDAL®, from Janssen); quetiapine fumarate (available under the tradename SEROQUEL®, from AstraZeneca); haloperidol (available under the tradename HALDOL®, from Ortho-McNeil); chlorpromazine (available under the tradename THORAZINE®, from SmithKline Beecham); fluphenazine (available under the tradename PROLIXIN®, from Apothecon, Copley, Schering, Teva, and American Pharmaceutical Partners, Pasadena); thiothixene (available under the tradename NAVANE®, from Pfizer); trifluoperazine (10-[3-(4-methyl-1-piperazinyl) propyl]-2-(trifluoromethyl) phenothiazine dihydrochloride, available under the tradename STELAZINE®, from SmithKlein Beckman); perphenazine (available under the tradename TRILAFON®, from Schering); thioridazine (available under the tradename MELLARIL®, from Novartis, Roxane, Hi-Tech, Teva, and Alpharma); molindone (available under the tradename MOBAN®, from Endo); and loxapine (available under the tradename LOXITANE® from Watson). Furthermore, benperidol (Glianimon®), perazine (Taxilan®) or melperone (Eunerpan®) may be used.

Other preferred neuroleptic drugs include promazine (available under the tradename SPARINE®), triflurpromazine (available under the tradename VESPRIN®), chlorprothixene (available under the tradename TARACTAN®), droperidol (available under the tradename INAPSINE®), acetophenazine (available under the tradename TINDAL®), prochlorperazine (available under the tradename COMPAZINE®), methotrimeprazine (available under the tradename NOZINAN®), pipotiazine (available under the tradename PIPOTRIL®), ziprasidone, and hoperidone.

Preferred neuroleptic drugs include risperidone and aripiprazole (from Bristol Myers Squibb Company, see e.g. Stahl SM; Dopamine-system stabilizers, aripiprazole and the next generation of antipsychotics, part I, "goldilocks"-actions at dopamine receptors; J. Clin. Psychiatry 2001, 62, 11:841-842).

The most preferred neuroleptic drug within the present invention is risperidone (Risperdal^{®}), its manufacture and pharmacological activity is described in EP 0 196 132. Risperidone acts as an antagonist to neurotransmitters, in particular dopamine, and is used for the treatment of psychoses.

Within the present invention, the neuroleptic risperidone can be administered at a dose of 2-6 mg/day, preferably 4-5 mg. The dose for celecoxib may range from 50-1600 mg, preferably 200-600, more preferably 400 mg. Preferably, the administration occurs twice daily (in the morning and in the evening).

Various types of antidepressants can be used for the add-on use according to the present invention. Examples of antidepressants that are useful in the present invention include: tricyclic antidepressants such as amitriptyline (5-(3-dimethylamino propylidene)-10,11-dihydro-5*H*-dibenzo[*a*,*d*]cyclohepten), amitriptyline oxide, desipramine (10,11-dihydro-5-(3-methylamino propyl)-5*H-*dibenz[*b*,*f*]azepin), dibenzepin (10-(2-dimethylamino ethyl)-5,11-dihydro-5-methyl-*11H*-dibenzo[*b,e*][1,4]diazepin-11-on), dosulepin (3-(6*H*-dibenzo[*b,e*]thiepin-11-yliden)-*N,N*- dimethylpropyl amine), doxepin (3-(6*H*-dibenz[*b,e*]oxepin-11-yliden)-dimethylpropyl amine), chloroimipramine, imipramine (5-(3-dimethylamino propyl)-5,11-dihydro-5*H* dibenz[*b,f*]azepin), nortriptyline (3-(10,11-dihydro-5*H-*dibenzo[*a,d*]cyclohepten-5-yliden)-*N*-methyl-1-propane amine), mianserin (1,2,3,4,10,14b-hexahydro-2-methyl-dibenzo[*c,f*]pyrazino[1,2-α]azepin), maprotiline (N-methyl-9,10-ethanoanthracene-9(10*H*)-propane amine), trimipramine (5-[3-dimethylamino)-2-methylpropyl]-10,11-dihydro-5*H*-dibenz[*b,f*]-azepin) or viloxazine (*R*S)-2-(2-ethyoxy phenoxy methyl)-morpholine), modem antidepressants such as trazodone (2-{3-[4-(3-chlorophenyl)-1-piperazinyl]-propyl}-1,2,4-triazolo[4,3-α,]pyridine-3(2*H*)-on, nefazodone (2-{3-[4-(3-chlorophenyl)-1-piperazinyl]propyl}-5-ethyl-2,4-dihydro-4-(2-phenoxyethyl)-3*H*-1,2,4-triazol-3-on), mirtazapine ((±)-1,2,3,4,10,14b-hexahydro-2-methylpyrazino[2,1-*a*]pyrido[2,3-*c*][2]benzazepin), venlafaxine ((±)-1-2-(dimethylamino)-1-(4-methoxyphenyl)-ethyl]cyclohexanol) or reboxetine ((±)-(2*RS*)-2-[(α*SR*)-α-(2-ethoxyphenoxy)benzy]morpholine), inhibitors of monoaminooxidases such as tranylcypromine (*trans*-2-phenyl cyclopropyl amine), brofaromine or moclobemide (4-chloro-*N*-(2-morpholinoethyl)-benzamide), selective inhibitors of serotonin-uptake such as citalopram, paroxetine, fluoxetine ((RS)-*N*-methyl-3-phenyl-3-[4-(trifluoromethyl)phenoxy]propyl amine, available under the tradename PROZAC®), fluvoxamine ((*E*)-5-methyoxy-4'-(trifluoromethyl)-valerophenon-O-(2-aminoethyl) oxime) or sertraline ((1*S-cis*)-(+)-4-(3,4-dichlorophenyl)-1,2,3,4-tetrahydro-*N*-methyl-1-naphthalinamine), and vegetable antidepressants such as Hypericum (St. John's wort).

The invention is also directed to kit-of-parts that is suitable for use in the treatment of the disorders as set forth in the claims, comprising a first dosage form comprising a neuroleptic agent or an antidepressant and a second dosage form comprising a COX-2 inhibitor as claimed, for simultaneous, separate or sequential administration.

According to a preferred embodiment, the dosage form comprising a neuroleptic agent or an antidepressant and the second dosage form comprising a COX-2 inhibitor are administered simultaneously.

The subject pharmaceutical kit-of-parts may be administered enterally (orally) or parenterally. Parenteral administration includes subcutaneous, intramuscular, intradermal, intramammary, intravenous, and other administrative methods known in the art. Enteral administration includes solution, tablets, sustained release capsules, enteric coated capsules, and syrups. Preferably the administration of a pharmaceutical kit comprising a COX-2 inhibitor and a neuroleptic or antidepressant occurs enterally (orally), in form of tablets.

The treatment of the claimed disorders with COX-2 inhibitors, alone or in combination with a neuroleptic or antidepressant, may occur in addition to further drug therapies. Thus, tranquilizers may be used for the treatment of agitation, anxiety or sleep disturbances. Preferably lorazepam is used, which belongs to the class of benzodiazepines.

In the following, the use of COX-2 inhibitors in the treatment of schizophrenia will be (not part of the invention) discussed in more detail with reference to a patient study. The results of the patient study, which are illustrative for the present invention, are graphically represented in the attached figures, which will be discussed in more detail in the following.

Figure 1 shows the comparison of the PANSS score during treatment with risperidone-celecoxib or risperidone-placebo.

Figure 2 shows the comparison of the PANSS negative score during treatment with risperidone-celecoxib or risperidone-placebo.

Figure 3 shows the comparison of the PANSS global score during treatment with risperidone-celecoxib or risperidone-placebo.

Figure 4 shows the plasma levels of risperidone and 9-OH-risperidone during treatment with risperidone-celecoxib or risperidone-placebo.

Figure 5 shows the biperiden and benzodiazepine use during treatment with risperidone-celecoxib or risperidone-placebo.

The study was performed as a single-center, double-blind, placebo-controlled, randomized, parallel-groupe valuation of the combination therapy with celecoxib and risperidone versus a monotherapy with risperidone and placebo in schizophrenic patients. The study included 50 patients fulfilling the criteria for the diagnosis of schizophrenia according to DSM IV (American Psychiatric Association (1994), Diagnostic and Statistical Manual of Mental Disorders, 1 st Edition, American Psychiatric Press, Washington DC), of whom 25 belonged to the risperidone-placebo and 25 to the risperidone-celecoxib group. No significant differences were present between the two patient groups were found with regard to age, sex, duration or severity of the disease or psychopathology, risperidone dose or risperidone-plasma levels.

The patients received 2-6 mg/day of risperidone (Risperdal®), and depending on to which group they belonged, 400 mg/day of celecoxib (2x200 mg Celebrex^{®} mornings and evenings) or placebo over 5 weeks after a brief wash-out period of earlier antipsychotic medication. During the wash-out period, a benzodiazepine preparation (mostly lorazepam) was prescribed, if necessary. Patients with agitation, anxiety, or sleeping problems were also medicated with lorazepam during the study.

The psychopathology of the patients was assessed using the positive and negative syndrome scale (PANSS) (Kay et al., Schizophr. Bull. 1987, 13:261-276).

The extrapyramidal side effects were assessed by the EPS scale (Simpson and Angus, Acta Psychiat. Scand. 1970 (Suppl.), 212). The use of biperiden was monitored as a possible indicator for side effects of the antipsychotic medication.

In order to exclude the chance that possible differences in the therapeutic effectiveness between the two groups might be due to non-compliance during the risperidone therapy or to differences in risperidone metabolism, the plasma levels of risperidone or 9-OH-risperidone were monitored during the study.

The statistics were performed according to the criterion of "last observation carried forward" (LOCF), i.e., the last PANSS scores of the patients who dropped out before the end of the study were carried forward to all subsequent observation days. For the comparison of the main efficacy parameter, the mean change in the PANSS between the two treatment groups, t-tests for independent samples were employed. With reference to the underlying hypothesis of a better outcome of the celecoxib-risperidone group, a significance of p < 0.05 was calculated in the one-tailed t-test and used as the basis for the estimation of the sample size (statistical power) and for the comparison of the groups. For all other comparisons, two-tailed t-tests were used.

At the start of the study, in the risperidone-celecoxib group (average age 35.9 ± 12.8 years), the PANSS total score was 71.8 ±17.1. the PANSS global score was 34.0 ± 8.5, the PANSS positive score was 19.0 ± 5.9 and the PANSS negative score was 18.7 ± 6.3. In the risperidone-placebo group (average age 35.5 ± 13.6 years), the PANSS total score was 75.4 ± 12.9, the PANSS global score was 37.2 ± 7.1, the PANSS positive score was 17.2 ± 4.6 and the PANSS negative score was 21.1 ± 5.5. Consequently, there was no significant difference in the PANSS total score or any of the subscales.

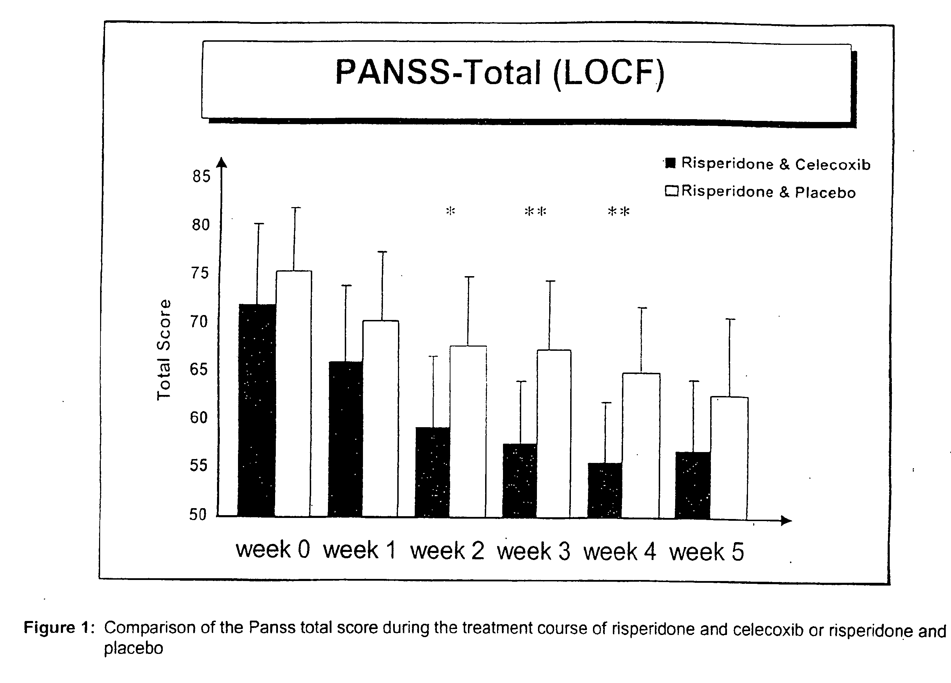

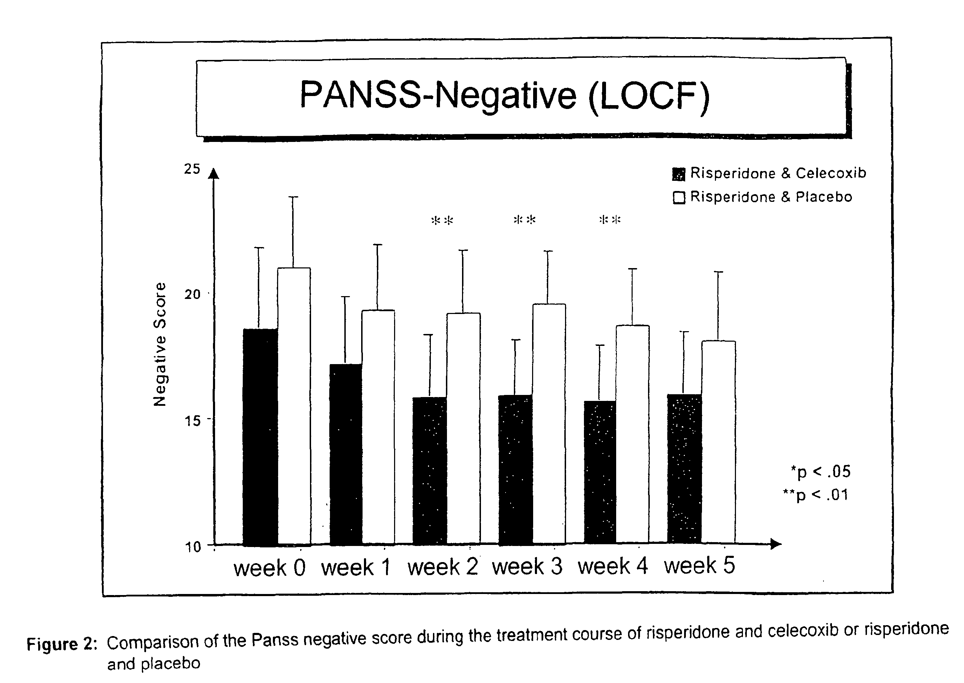

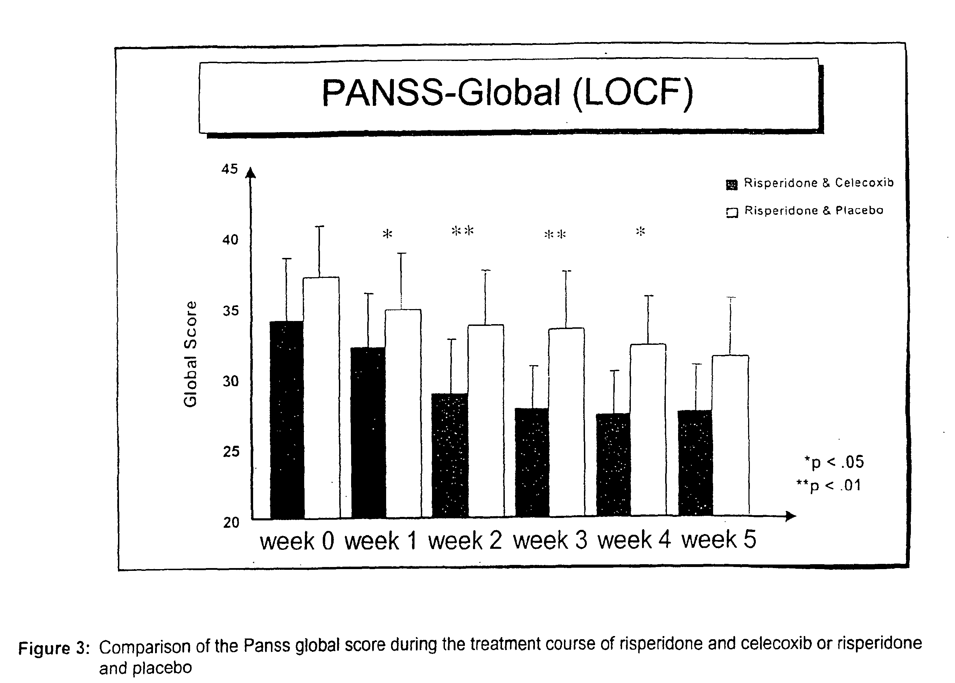

During the five-week therapy, a significant improvement of the PANSS total score and the subscales is observed in both groups of schizophrenic patients. The results of the PANSS total score are shown in Figure 1, of the PANSS negative score in Figure 2, of the PANSS global score in Figure 3 and of the PANSS positive score in Table 3.

**Table 3**

| Comparison of the PANSS positive score | | | | |
|---|---|---|---|---|
| time | celecoxib and risperidone | placebo and risperidone | t¹ | ρ² |
| week 0 | 19.0 ± 5.9 | 17.2 ± 4.6 | 1.22 | n.s.³ |
| week 1 | 16.7 ± 5,5 | 16.2 ± 4.6 | 0.36 | n.s. |
| week 2 | 14.4 ± 5.0 | 15 ± 4.5 | 0.42 | n.s. |
| week 3 | 14.0 ± 4.7 | 14.5 ± 4.6 | 0.36 | n.s. |
| week 4 | 12.8 ± 4.4' | 14.2 ± 4.4 | 1.16 | n.s. |
| week 5 | 13.4 ± 5.6 | 13.3 ± 4.4 | 0.11 | n.s. |

| | | | | |
|---|---|---|---|---|
| ¹ t represents the statistical random sample distribution. ² p represents the statistical power (probability). ³ n.s. means no statistical significance. | | | | |

In the celecoxib-risperidone group, the two-tailed t-tests between the baseline and week 5 gave the following values: PANSS total score p < 0.0001, PANSS global score p < 0.0001, PANSS positive score p < 0.0001, PANSS negative score p < 0.001. In the placebo-risperidone group, the t-tests between the baseline and week 5 gave the following values: PANSS total score p < 0.002, PANSS global score p < 0.003, PANSS positive score p < 0.002, PANSS negative score p < 0.02.

The improved effectiveness of the combination therapy with celecoxib-risperidone in comparison to risperidone monotherapy is clearly shown by the significantly lower PANSS global scores after the 2, 3, 4 and 5 weeks of treatment (Figure 3). With regard to the total and negative score, significantly lower scores were recorded after 2, 3 and 4 weeks in the celecoxib-risperidone group (Figures 1 and 2).

The mean daily dose of risperidone is shown in Table 4; no statistically significant difference was found between the two treatment groups.

**Table 4**

| Mean risperidone dose mg/day | | | |
|---|---|---|---|
| time | celecoxib and risperidone | placebo and risperidone | difference |
| week1 | 4.1 ± 0.6 | 4.0 ± 0.8 | n.s. |
| week 2 | 4.5 ± 0.6 | 4.4 ± 1.1 | n.s. |
| week 3 | 4.8 ± 0.8 | 4.9 ± 1.4 | n.s. |
| week 4 | 5.0 ± 1.0 | 4.9 ± 1.4 | n.s. |
| week 5 | 4.9 ± 1.0 | 5.1 ± 1.5 | n.s. |

| | | | |
|---|---|---|---|
| ¹ n.s. means no statistical significance. | | | |

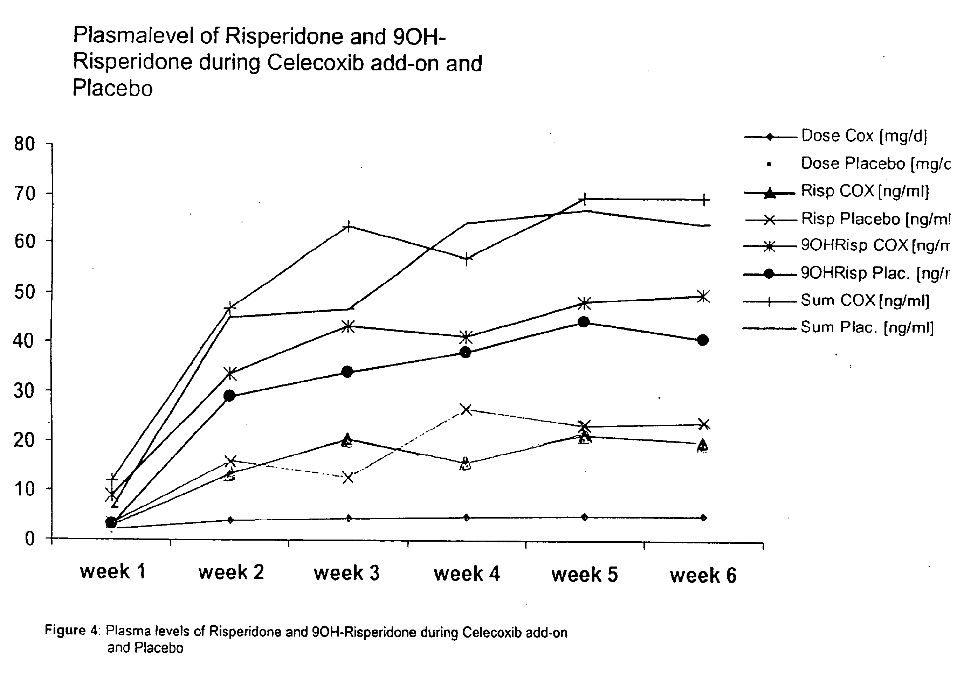

The differences in the plasma levels of risperidone or the metabolite 9-OH-risperidone shown in Figure 4 were also without statistical significance ( the present Figure 4 differs from Figure 4 of the German patent application priority document due to a calculation error in said priority document).
Therefore, it could be excluded that the observed differences in the therapeutic effectiveness between the two groups are due to incompatibility during the risperidone therapy or differences in risperidone metabolism. The therapeutic benefit of the combined therapy has to be attributed to the COX-2 inhibitor, celecoxib.

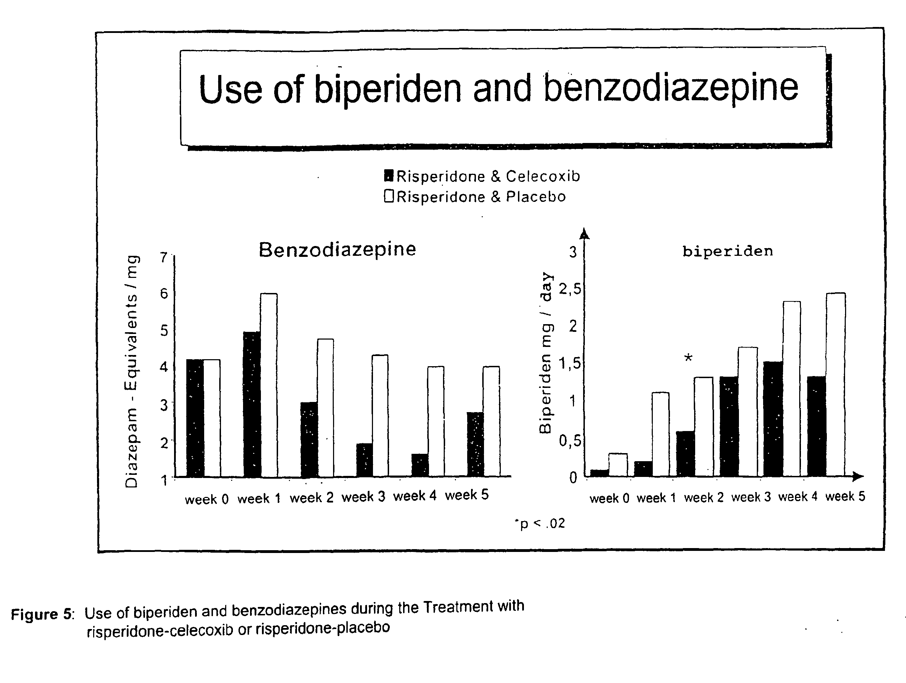

With respect to the extrapyramidal side effects, no statistically significant differences were found in the EPS scale. The use of biperiden is shown in Figure 5 and was calculated as cumulative weekly dose. The values were lower in the celecoxib-risperidone group, and reached statistical significance at week 2 (p < 0.02).

A detailed analysis of items of the PANSS-Scale which discriminate good celecoxib-responders from the placebo group revealed that therapeutic effects of celecoxib are especially found on the items "lack of contact" (item 3 of the negative subscale), "emotional isolation" (item 2 of the negative subscale), "passive-apathic isolation" (item 4 of the negative subscale), "social withdrawal" (item 16 of the general psychopathology subscale), "depression" (item 6 of the general psychopathology subscale) and "motor retardation" (item 6 of the general psychopathology subscale).

Furthermore, a factor analysis showed that especially items which can subsumed under the label "agitation" show a good therapeutic response to celecoxib, but not to placebo. All those items reflect psychopathological symptoms which are typically found in depressive states. Therefore this detailed analysis points to a therapeutic efficiency in depressive states.

Moreover, "passive-apathic isolation", "motor retardation", "social withdrawal", or "lack of contact" are -often more severe expressed than in depressive states - also core-symptoms of childhood autism.

The combination of celecoxib and risperidone according to the present invention thus shows improved results compared to the monopreparation risperidone with regard to effectiveness in the treatment of schizophrenia. Furthermore, it was observed that the beneficial effects of the add-on therapy occurred faster in patients with a recent onset of the disorder and that the celecoxib therapy was useful in the treatment of depressive states.

## Claims

1. Use of a tricyclic COX-2 inhibitor of the formula: 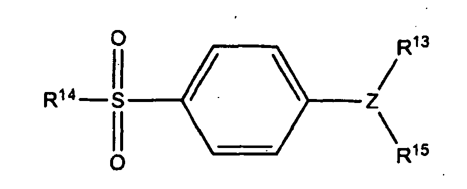 wherein:
Z is selected from the group consisting of partially unsaturated or unsaturated heterocyclyl and partially unsaturated or unsaturated carbocyclic rings;
R¹³ is selected from the group consisting of heterocyclyl, cycloalkyl, cycloalkenyl and aryl, wherein R¹³ is optionally substituted at a substitutable position with one or more radicals selected from alkyl, haloalkyl, cyano, carboxyl, alkoxycarbonyl, hydroxyl, hydroxyalkyl, haloalkoxy, amino, alkylamino, arylamino, nitro, alkoxyalkyl, alkylsulfinyl, halo, alkoxy and alkylthio;
R¹⁴ is selected from the group consisting of methyl or amino; and
R¹⁵ is selected from the group consisting of a radical selected from H, halo, alkyl, alkenyl, alkynyl, oxo, cyano, carboxyl, cyanoalkyl, heterocyclyloxy, alkyloxy, alkylthio, alkylcarbonyl, cycloalkyl, aryl, haloalkyl, heterocyclyl, cycloalkenyl, aralkyl, heterocyclylalkyl, acyl, alkylthioalkyl, hydroxyalkyl, alkoxycarbonyl, arylcarbonyl, aralkylcarbonyl, aralkenyl, alkoxyalkyl, arylthioalkyl, aryloxyalkyl, aralkylthioalkyl, aralkoxyalkyl, alkoxyaralkoxyalkyl, alkoxycarbonylalkyl, aminocarbonyl, aminocarbonylalkyl, alkylaminocarbonyl, N-arylaminocarbonyl, N-alkyl-N-arylaminocarbonyl, alkylaminocarbonylalkyl, carboxyalkyl, alkylamino, N-arylamino, N-aralkylamino, N-alkyl-N-aralkylamino, N-alkyl-N-arylamino, aminoalkyl, alkylaminoalkyl, N-arylaminoalkyl, N-aralkylaminoalkyl, N-alkyl-N-aralkylaminoalkyl, N-alkyl-N-arylaminoalkyl, aryloxy, aralkoxy, arylthio, aralkylthio, alkylsulfinyl, alkylsulfonyl, aminosulfonyl, alkylaminosulfonyl, N-arylaminosulfonyl, arylsulfonyl, N-alkyl-N-arylaminosulfonyl;
in the preparation of a medicament for the treatment of depression, depressive episodes, recurring depressive episodes, manic episodes and bipolar affective disorders.

2. Use according to Claims 1, **characterized in that** the COX-2 inhibitor is selected from celecoxib, rofecoxib, deracoxib, parecoxib, valdecoxib, etoricoxib, ABT963 or JTE-522, pharmaceutically acceptable salts, or mixtures thereof.

3. Use according to Claim 2, **characterized in that** celecoxib or a pharmaceutically acceptable salt thereof is used as COX-2 inhibitor.

4. Use according to Claim 3, **characterized in that** celecoxib or a pharmaceutically acceptable salt thereof is to be administered in an amount of 50-1600 mg per day, preferably 200 to 600 mg, most preferably 400 mg.

5. Use according to anyone of the preceding Claims, **characterized in that** the medicament is to be administered orally.

6. Use of a tricyclic COX-2 inhibitor represented by the general structure of formula: 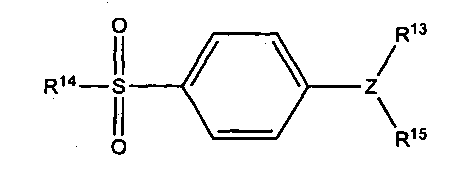 wherein:
Z is selected from the group consisting of partially unsaturated or unsaturated heterocyclyl and partially unsaturated or unsaturated carbocyclic rings;
R¹³ is selected from the group consisting of heterocyclyl, cycloalkyl, cycloalkenyl and aryl, wherein R¹³ is optionally substituted at a substitutable position with one or more radicals selected from alkyl, haloalkyl, cyano, carboxyl, alkoxycarbonyl, hydroxyl, hydroxyalkyl, haloalkoxy, amino, alkylamino, arylamino, nitro, alkoxyalkyl, alkylsulfinyl, halo, alkoxy and alkylthio;
R¹⁴ is selected from the group consisting of methyl or amino; and
R¹⁵ is selected from the group consisting of a radical selected from H, halo, alkyl, alkenyl, alkynyl, oxo, cyano, carboxyl, cyanoalkyl, heterocyclyloxy, alkyloxy, alkylthio, alkylcarbonyl, cycloalkyl, aryl, haloalkyl, heterocyclyl, cycloalkenyl, aralkyl, heterocyclylalkyl, acyl, alkylthioalkyl, hydroxyalkyl, alkoxycarbonyl, arylcarbonyl, aralkylcarbonyl, aralkenyl, alkoxyalkyl, arylthioalkyl, aryloxyalkyl, aralkylthioalkyl, aralkoxyalkyl, alkoxyaralkoxyalkyl, alkoxycarbonylalkyl, aminocarbonyl, aminocarbonylalkyl, alkylaminocarbonyl, N-arylaminocarbonyl, N-alkyl-N-arylaminocarbonyl, alkylaminocarbonylalkyl, carboxyalkyl, alkylamino, N-arylamino, N-aralkylamino, N-alkyl-N-aralkylamino, N-alkyl-N-arylamino, aminoalkyl, alkylaminoalkyl, N-arylaminoalkyl, N-aralkylaminoalkyl, N-alkyl-N-aralkylaminoalkyl, N-alkyl-N-arylaminoalkyl, aryloxy, aralkoxy, arylthio, aralkylthio, alkylsulfinyl, alkylsulfonyl, aminosulfonyl, alkylaminosulfonyl, N-arylaminosulfonyl, arylsulfonyl, N-alkyl-N-arylaminosulfonyl;
in combination with a neuroleptic drug or an antidepressant in the preparation of a medicament or medicaments for the treatment of depressive episodes, recurring depressive episodes, manic episodes and bipolar affective disorders.

7. Use according to Claim 6, **characterized in that** the COX-2 inhibitor is selected from celecoxib, rofecoxib, deracoxib, parecoxib, valdecoxib, etoricoxib, ABT963 or JTE-522, pharmaceutically acceptable salts, or mixtures thereof.

8. Use according to Claim 7, **characterized in that** celecoxib or a pharmaceutically acceptable salt thereof is used as COX-2 inhibitor.

9. Use according to anyone of Claims 6 to 8, **characterized in that** the neuroleptic is selected from clozapine, olanzapine, ziprasidone, risperidone, aripiprazole, quetiapine, quetiapine fumarate, sertindole, amisulpride, haloperidol, haloperidol decanoate, haloperidol lactate, chlorpromazine, fluphenazine, fluphenazine decanoate, fluphenazine enanthate, fluphenazine hydrochloride, thiothixene, thiothixene hydrochloride, trifluoperazine, perphenazine, amitriptyline, thioridazine, mesoridazine, molinfone, molindone hydrochloride, loxapine, loxapine hydrochloride, loxapine succinate, pimozide, flupenthixol, promazine, triflupromazine, chlorprothixene, droperidol, actophenazine, prochlorperazine, methotrimeprazine, pipotiazine, ziprasidone, hoperidone, zuclopenthixol, and mixtures thereof.

10. Use according to anyone of Claims 6 to 8, **characterized in that** the antidepressant is selected from amitriptyline, amitriptyline oxide, desipramine, dibenzepin, dosulepin, doxepin, chloroimipramine, imipramine, nortriptyline, mianserin, maprotiline, trimipramine, viloxazine, trazodone, nefazodone, mirtazapine, venlafaxine_{;} reboxetine, tranylcypromine, brofaromine, moclobemide, citalopram, paroxetine, fluoxetine, fluvoxamine, sertraline, Hypericum (St. John's Wort), and mixtures thereof.

11. Use according to claim 9, **characterized in that** risperidone or aripiprazole is used as a neuroleptic.

12. Use according to Claim 11, **characterized in that** celecoxib or a pharmaceutically acceptable salt thereof and risperidone are to be administered in an amount of 50-1600 mg, preferably 200-600 mg, and 2-6 mg, respectively.

13. Use according to Claim 12, **characterized in that** celecoxib or a pharmaceutically acceptable salt thereof and risperidone are to be administered in an amount of 400 mg and 4-5 mg, respectively.

14. Use according to claim 10, **characterized in that** reboxetine is used as antidepressant.

15. Use according to anyone of Claims 6 to 14, **characterized in that** the medicament is to be administered orally.

16. Use according to anyone of the preceding Claims, **characterized in that** a tranquilizer, preferably lorazepam, is to be administered additionally.

17. Kit-of-parts suitable for use in the treatment of depression, depressive episodes, recurring depressive episodes, manic episodes and bipolar affective disorders, comprising a first dosage form comprising a neuroleptic drug or an antidepressant and a second dosage form comprising a tricyclic COX-2 inhibitor represented by the general structure of formula: 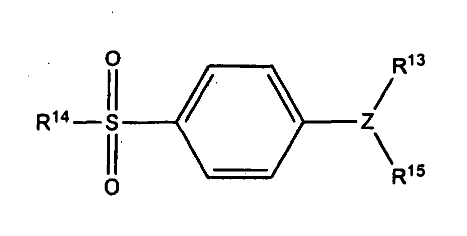 wherein:
Z is selected from the group consisting of partially unsaturated or unsaturated heterocyclyl and partially unsaturated or unsaturated carbocyclic rings;
R¹³ is selected from the group consisting of heterocyclyl, cycloalkyl, cycloalkenyl and aryl, wherein R¹³ is optionally substituted at a substitutable position with one or more radicals selected from alkyl, haloalkyl, cyano, carboxyl, alkoxycarbonyl, hydroxyl, hydroxyalkyl, haloalkoxy, amino, alkylamino, arylamino, nitro, alkoxyalkyl, alkylsulfinyl, halo, alkoxy and alkylthio;
R¹⁴ is selected from the group consisting of methyl or amino; and
R¹⁵ is selected from the group consisting of a radical selected from H, halo, alkyl, alkenyl, alkynyl, oxo, cyano, carboxyl, cyanoalkyl, heterocyclyloxy, alkyloxy, alkylthio, alkylcarbonyl, cycloalkyl, aryl, haloalkyl, heterocyclyl, cycloalkenyl, aralkyl, heterocyclylalkyl, acyl, alkylthioalkyl, hydroxyalkyl, alkoxycarbonyl, arylcarbonyl, aralkylcarbonyl, aralkenyl, alkoxyalkyl, arylthioalkyl, aryloxyalkyl, aralkylthioalkyl, aralkoxyalkyl, alkoxyaralkoxyalkyl, alkoxycarbonylalkyl, aminocarbonyl, aminocarbonylalkyl, alkylaminocarbonyl, N-arylaminocarbonyl, N-alkyl-N-arylaminocarbonyl, alkylaminocarbonylalkyl, carboxyalkyl, alkylamino, N-arylamino, N-aralkylamino, N-alkyl-N-aralkylamino, N-alkyl-N-arylamino, aminoalkyl, alkylaminoalkyl, N-arylaminoalkyl, N-aralkylaminoalkyl, N-alkyl-N-aralkylaminoalkyl, N-alkyl-N-arylaminoalkyl, aryloxy, aralkoxy, arylthio, aralkylthio, alkylsulfinyl, alkylsulfonyl, aminosulfonyl, alkylaminosulfonyl, N-arylaminosuffonyl, arylsulfonyl, N-alkyl-N-arylaminosulfonyl;
for simultaneous, separate or sequential administration.

18. Kit-of-parts according to Claim 17, **characterized in that** the neuroleptic is selected from clozapine, alanzapine, ziprasidone, risperidone, quetiapine, quetiapine fumarate, sertinfole, amisulpride, haloperidol, haloperidol decanoate, haloperidol lactate, chlorpromazine, fluphenazine, fluphenazine decanoate, fluphenazine enanthate, fluphenazine hydrochloride, thiothixene, thiothixene hydrochloride, trifluoperazine, perphenazine, amitriptyline, thioridazine, mesoridazine, molindone, molindone hydrochloride, loxapine, loxapine hydrochloride, loxapine succinate, pimozide, flupenthixol, promazine, triflupromazine, chlorprothixene, droperidol, actophenazine, prochlorperazine, methotrimeprazine, pipotiazine, ziprasidone, hoperidone, zuclopenthixol, and mixtures thereof.

19. Kit-of-parts according to, Claim 17, **characterized in that** the antidepressant is selected from amitriptyline, amitriptyline oxide, desipramine, dibenzepin, dosulepin; doxepin, chloroimipramine, imipramine, nortriptyline, mianserin, maprotiline, trimipramine, viloxazine, trazodone, nefazodone, mirtazapine, venlafaxine, reboxetine, tranylcypromine, brofaromine, moclobemide, citalopram, paroxetine, fluoxetine, fluvoxamine, sertraline, Hypericum (St. John's Wort), and mixtures thereof.

20. Kit-of-parts according to anyone of Claims 17 to 19, **characterized in that** the COX-2 inhibitor is selected from celecoxib, rofecoxib, deracoxib, parecoxib, valdecoxib, etoricoxib, ABT963 or JTE-522, pharmaceutically acceptable salts, or mixtures thereof.

21. Kit-of-parts according to claim 18, **characterized by** comprising celecoxib or a pharmaceutically acceptable salt thereof and risperidone as neuroleptic drug.

22. Kit-of-parts according to claim 19, **characterized by** comprising celecoxib or a pharmaceutically acceptable salt thereof and reboxetine as antidepressant.

## Patentansprüche

1. Verwendung eines tricylischen COX-2-Inhibitors der Formel: 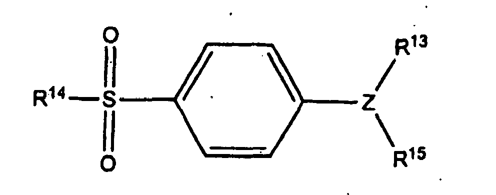 wobei:
Z aus der Gruppe bestehend aus partiell ungesättigten oder ungesättigten heterocyclischen und partiell ungesättigten oder ungesättigten carbocyclischen Ringen ausgewählt ist;
R¹³ aus der Gruppe bestehend aus Heterocyclyl, Cycloalkyl, Cycloalkenyl und Aryl ausgewählt ist, wobei R¹³ gegebenenfalls an einer substituierbaren Stelle mit einem oder mehreren Resten ausgewählt aus Alkyl, Halogenalkyl, Cyano, Carboxyl, Alkoxycarbonyl, Hydroxyl, Hydroxyalkyl, Halogenalkoxy, Amino, Alkylamino, Arylamino, Nitro, Alkoxyalkyl, Alkylsulfinyl, Halogen, Alkoxy und Alkylthio substituiert ist;
R¹⁴ aus der Gruppe bestehend aus Methyl oder Amino ausgewählt ist; und
R¹⁵ ausgewählt ist aus der Gruppe bestehend aus einem Rest ausgewählt aus H, Halogen, Alkyl, Alkenyl, Alkinyl, Oxo, Cyano, Carboxyl, Cyanoalkyl, Heterocyclyloxy, Alkyloxy, Alkylthio, Alkylcarbonyl, Cycloalkyl, Aryl, Halogenalkyl, Heterocyclyl, Cycloalkenyl, Aralkyl, Heterocyclylalkyl, Acyl, Alkylthioalkyl, Hydroxyalkyl, Alkoxycarbonyl, Arylcarbonyl, Aralkylcarbonyl, Aralkenyl, Alkoxyalkyl, Arylthioalkyl, Aryloxyalkyl, Aralkylthioalkyl, Aralkoxyalkyl, Alkoxyaralkoxyalkyl, Alkoxycarbonylalkyl, Aminocarbonyl, Aminocarbonylalkyl, Alkylaminocarbonyl, N-Arylaminocarbonyl, N-Alkyl-N-arylaminocarbonyl, Alkylaminocarbonylalkyl, Carboxyalkyl, Alkylamino, N-Arylamino, N-Aralkylamino, N-Alkyl-N-aralkylamino, N-Alkyl-N-arylamino, Aminoalkyl, Alkylaminoalkyl, N-Arylaminoalkyl, N-Aralkylaminoalkyl, N-Alkyl-N-aralkylaminoalkyl, N-Alkyl-N-arylaminoalkyl, Aryloxy, Aralkoxy, Arylthio, Aralkylthio, Alkylsulfinyl, Alkylsulfonyl, Aminosulfonyl, Alkylaminosulfonyl, N-Arylaminosulfonyl, Arylsulfonyl, N-Alkyl-N-arylaminosulfonyl;
zur Herstellung eines Medikaments zur Behandlung von Depression, depressiven Episoden, wiederkehrenden depressiven Episoden, manischen Episoden und bipolaren affektiven Störungen.

2. Verwendung nach Anspruch 1, **dadurch gekennzeichnet, dass** der COX-2-Inhibitor aus Celecoxib, Rofecoxib, Deracoxib, Parecoxib, Valdecoxib, Etoricoxib, ABT963 oder JTE-522, pharmazeutisch verträglichen Salzen oder Mischungen davon ausgewählt ist.

3. Verwendung nach Anspruch 2, **dadurch gekennzeichnet, dass** als COX-2-Inhibitor Celecoxib oder ein pharmazeutisch verträgliches Salz davon verwendet wird.

4. Verwendung nach Anspruch 3, **dadurch gekennzeichnet, dass** Celecoxib oder ein pharmazeutisch verträgliches Salz davon in einer Menge von 50-1600 mg pro Tag, vorzugsweise 200 bis 600 mg, am meisten bevorzugt 400 mg, zu verabreichen ist.

5. Verwendung nach einem der vorhergehenden Ansprüche, **dadurch gekennzeichnet, dass** das Medikament oral zu verabreichen ist.

6. Verwendung eines tricyclischen COX-2-Inhibitors, der durch die allgemeine Struktur der Formel: 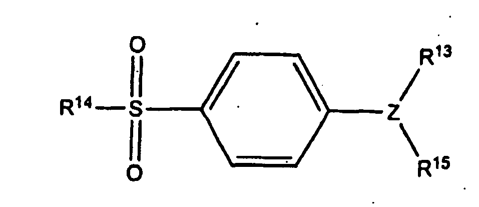 dargestellt ist, wobei:
Z aus der Gruppe bestehend aus partiell ungesättigten oder ungesättigten heterocyclischen und partiell ungesättigten oder ungesättigten carbocyclischen Ringen ausgewählt ist;
R1³ aus der Gruppe bestehend aus Heterocyclyl, Cycloalkyl, Cycloalkenyl und Aryl ausgewählt ist, wobei R¹³ gegebenenfalls an einer substituierbaren Stelle mit einem oder mehreren Resten ausgewählt aus Alkyl, Halogenalkyl, Cyano, Carboxyl, Alkoxycarbonyl, Hydroxyl, Hydroxyalkyl, Halogenalkoxy, Amino, Alkylamino, Arylamino, Nitro, Alkoxyalkyl, Alkylsulfinyl, Halogen, Alkoxy und Alkylthio substituiert ist;
R¹⁴ aus der Gruppe bestehend aus Methyl oder Amino ausgewählt ist; und
R¹⁵ ausgewählt ist aus der Gruppe bestehend aus einem Rest ausgewählt aus H, Halogen, Alkyl, Alkenyl, Alkinyl, Oxo, Cyano, Carboxyl, Cyanoalkyl, Heterocyclyloxy, Alkyloxy, Alkylthio, Alkylcarbonyl, Cycloalkyl, Aryl, Halogenalkyl, Heterocyclyl, Cycloalkenyl, Aralkyl, Heterocyclylalkyl, Acyl, Alkylthioalkyl, Hydroxyalkyl, Alkoxycarbonyl, Arylcarbonyl, Aralkylcarbonyl, Aralkenyl, Alkoxyalkyl, Arylthioalkyl, Aryloxyalkyl, Aralkylthioalkyl, Aralkoxyalkyl, Alkoxyaralkoxyalkyl, Alkoxycarbonylalkyl, Aminocarbonyl, Aminocarbonylalkyl, Alkylaminocarbonyl, N-Arylaminocarbonyl, N-Alkyl-N-arylaminocarbonyl, Alkylaminocarbonylalkyl, Carboxyalkyl, Alkylamino, N-Arylamino, N-Aralkylamino, N-Alkyl-N-aralkylamino, N-Alkyl-N-arylamino, Aminoalkyl, Alkylaminoalkyl, N-Arylaminoalkyl, N-Aralkylaminoalkyl, N-Alkyl-N-aralkylaminoalkyl, N-Alkyl-N-arylaminoalkyl, Aryloxy, Aralkoxy, Arylthio, Aralkylthio, Alkylsulfinyl, Alkylsulfonyl, Aminosulfonyl, Alkylaminosulfonyl, N-Arylaminosulfonyl, Arylsulfonyl, N-Alkyl-N-arylaminosulfonyl;
in Kombination mit einem Neuroleptikum oder einem Antidepressivum zur Herstellung eines Medikaments oder von Medikamenten zur Behandlung von depressiven Episoden, wiederkehrenden depressiven Episoden, manischen Episoden und bipolaren affektiven Störungen.

7. Verwendung nach Anspruch 6, **dadurch gekennzeichnet, dass** der COX-2-Inhibitor aus Celecoxib, Rofecoxib, Deracoxib, Parecoxib, Valdecoxib, Etoricoxib, ABT963 oder JTE-522, pharmazeutisch verträglichen Salzen oder Mischungen davon ausgewählt ist.

8. Verwendung nach Anspruch 7, **dadurch gekennzeichnet, dass** als COX-2-Inhibitor Celecoxib oder ein pharmazeutisch verträgliches Salz davon verwendet wird.

9. Verwendung nach einem der Ansprüche 6 bis 8, **dadurch gekennzeichnet, dass** das Neuroleptikum aus Clozapin, Olanzapin, Ziprasidon, Risperidon, Aripiprazol, Quetiapin, Quetiapinfumarat, Sertindol, Amisulprid, Haloperidol, Haloperidoldecanoat, Haloperidollactate, Chlorpromazin, Fluphenazin, Fluphenazindecanoat, Fluphenazinenanthat, Fluphenazinhydrochlorid, Thiothixen, Thiothixenhydrochlorid, Trifluoperazin, Perphenazin, Amitriptylin, Thioridazin, Mesoridazin, Molindon, Molindonhydrochlorid, Loxapin, Loxapinhydrochlorid, Loxapinsuccinat, Pimozid, Flupenthixol, Promazin, Triflupromazin, Chlorprothixen, Droperidol, Acetophenazin, Prochlorperazin, Methotrimeprazin, Pipotiazin, Ziprasidon, Hoperidon, Zuclopenthixol, und Mischungen davon ausgewählt ist.

10. Verwendung nach einem der Ansprüche 6 bis 8, **dadurch gekennzeichnet, dass** das Antidepressivum aus Amitriptylin, Amitriptylinoxid, Desipramin, Dibenzepin, Dosulepin, Doxepin, Chlorimipramin, Imipramin, Nortriptylin, Mianserin, Maprotilin, Trimipramin, Viloxazin, Trazodon, Nefazodon, Mirtazapin, Venlafaxin, Reboxetin, Tranylcypromin, Brofaromin, Moclobemid, Citalopram, Paroxetin, Fluoxetin, Fluvoxamin, Sertralin, Hypericum (Johanniskraut), und Mischungen davon ausgewählt ist.

11. Verwendung nach Anspruch 9, **dadurch gekennzeichnet, dass** Risperidon oder Aripiprazol als Neuroleptikum verwendet wird.

12. Verwendung nach Anspruch 11, **dadurch gekennzeichnet, dass** Celecoxib oder ein pharmazeutisch verträgliches Salz davon und Risperidon in einer Menge von 50-1600 mg, vorzugsweise 200-600 mg, beziehungsweise 2-6 mg zu verabreichen sind.

13. Verwendung nach Anspruch 12, **dadurch gekennzeichnet, dass** Celecoxib oder ein pharmazeutisch verträgliches Salz davon und Risperidon in einer Menge von 400 mg beziehungsweise 4-5 mg zu verabreichen sind.

14. Verwendung nach Anspruch 10, **dadurch gekennzeichnet, dass** Reboxetin als Antidepressivum verwendet wird.

15. Verwendung nach einem der Ansprüche 6 bis 14, **dadurch gekennzeichnet, dass** das Medikament oral zu verabreichen ist.

16. Verwendung nach einem der vorhergehenden Ansprüche, **dadurch gekennzeichnet, dass** zusätzlich ein Beruhigungsmittel, vorzugsweise Lorazepam, zu verabreichen ist.

17. Kit-of-Parts, das zur Verwendung in der Behandlung von Depression, depressiven Episoden, wiederkehrenden depressiven Episoden, manischen Episoden und bipolaren affektiven Störungen geeignet ist, umfassend eine erste Dosierungsform, die ein Neuroleptikum oder ein Antidepressivum umfasst, und eine zweite Dosierungsform, die einen tricyclischen COX-2-Inhibitor umfasst, der durch die allgemeine Struktur der Formel: 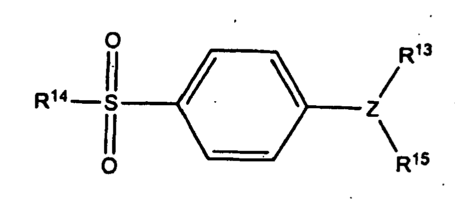 dargestellt ist, wobei:
Z aus der Gruppe bestehend aus partiell ungesättigten oder ungesättigten heterocyclischen und partiell ungesättigten oder ungesättigten carbocyclischen Ringen ausgewählt ist;
R¹³ aus der Gruppe bestehend aus Heterocyclyl, Cycloalkyl, Cycloalkenyl und Aryl ausgewählt ist, wobei R¹³ gegebenenfalls an einer substituierbaren Stelle mit einem oder mehreren Resten ausgewählt aus Alkyl, Halogenalkyl, Cyano, Carboxyl, Alkoxycarbonyl, Hydroxyl, Hydroxyalkyl, Halogenalkoxy, Amino, Alkylamino, Arylamino, Nitro, Alkoxyalkyl, Alkylsulfinyl, Halogen, Alkoxy und Alkylthio substituiert ist;
R¹⁴ aus der Gruppe bestehend aus Methyl oder Amino ausgewählt ist; und
R¹⁵ ausgewählt ist aus der Gruppe bestehend aus einem Rest ausgewählt aus H, Halogen, Alkyl, Alkenyl, Alkinyl, Oxo, Cyano, Carboxyl, Cyanoalkyl, Heterocyclyloxy, Alkyloxy, Alkylthio, Alkylcarbonyl, Cycloalkyl, Aryl, Halogenalkyl, Heterocyclyl, Cycloalkenyl, Aralkyl, Heterocyclylalkyl, Acyl, Alkylthioalkyl, Hydroxyalkyl, Alkoxycarbonyl, Arylcarbonyl, Aralkylcarbonyl, Aralkenyl, Alkoxyalkyl, Arylthioalkyl, Aryloxyalkyl, Aralkylthioalkyl, Aralkoxyalkyl, Alkoxyaralkoxyalkyl, Alkoxycarbonylalkyl, Aminocarbonyl, Aminocarbonylalkyl, Alkylaminocarbonyl, N-Arylaminocarbonyl, N-Alkyl-N-arylaminocarbonyl, Alkylaminocarbonylalkyl, Carboxyalkyl, Alkylamino, N-Arylamino, N-Aralkylamino, N-Alkyl-N-aralkylamino, N-Alkyl-N-arylamino, Aminoalkyl, Alkylaminoalkyl, N-Arylaminoalkyl, N-Aralkylaminoalkyl, N-Alkyl-N-aralkylaminoalkyl, N-Alkyl-N-arylaminoalkyl, Aryloxy, Aralkoxy, Arylthio, Aralkylthio, Alkylsulfinyl, Alkylsulfonyl, Aminosulfonyl, Alkylaminosulfonyl, N-Arylaminosulfonyl, Arylsulfonyl, N-Alkyl-N-arylaminosulfonyl;
für die gleichzeitige, getrennte oder sequenzielle Verabreichung.

18. Kit-of-Parts nach Anspruch 17, **dadurch gekennzeichnet, dass** das Neuroleptikum aus Clozapin, Olanzapin, Ziprasidon, Risperidon, Quetiapin, Quetiapinfumarat, Sertindol, Amisulprid, Haloperidol, Haloperidoldecanoat, Haloperidollactate, Chlorpromazin, Fluphenazin, Fluphenazindecanoat, Fluphenazinenanthat, Fluphenazinhydrochlorid, Thiothixen, Thiothixenhydrochlorid, Trifluoperazin, Perphenazin, Amitriptylin, Thioridazin, Mesoridazin, Molindon, Molindonhydrochlorid, Loxapin, Loxapinhydrochlorid, Loxapinsuccinat, Pimozid, Flupenthixol, Promazin, Triflupromazin, Chlorprothixen, Droperidol, Acetophenazin, Prochlorperazin, Methotrimeprazin, Pipotiazin, Ziprasidon, Hoperidon, Zuclopenthixol, und Mischungen davon ausgewählt ist.

19. Kit-of-Parts nach Anspruch 17, **dadurch gekennzeichnet, dass** das Antidepressivum aus Amitriptylin, Amitriptylinoxid, Desipramin, Dibenzepin, Dosulepin, Doxepin, Chlorimipramin, Imipramin, Nortriptylin, Mianserin, Maprotilin, Trimipramin, Viloxazin, Trazodon, Nefazodon, Mirtazapin, Venlafaxin, Reboxetin, Tranylcypromin, Brofaromin, Moclobemid, Citalopram, Paroxetin, Fluoxetin, Fluvoxamin, Sertralin, Hypericum (Johanniskraut), und Mischungen davon ausgewählt ist.

20. Kit-of-Parts nach einem dem Ansprüche 17 bis 19, **dadurch gekennzeichnet, dass** der COX-2-Inhibitor aus Celecoxib, Rofecoxib, Deracoxib, Parecoxib, Valdecoxib, Etoricoxib, ABT963 oder JTE-522, pharmazeutisch verträglichen Salzen oder Mischungen davon ausgewählt ist.

21. Kit-of-Parts nach Anspruch 18, **dadurch gekennzeichnet, dass** dieses Celecoxib oder ein pharmazeutisch verträgliches Salz davon und Risperidon als Neuroleptikum umfasst.

22. Kit-of-Parts nach Anspruch 19, **dadurch gekennzeichnet, dass** dieses Celecoxib oder ein pharmazeutisch verträgliches Salz davon und Reboxetin als Antidepressivum umfasst.

## Revendications

1. Utilisation d'un inhibiteur de COX-2 tricyclique de formule : 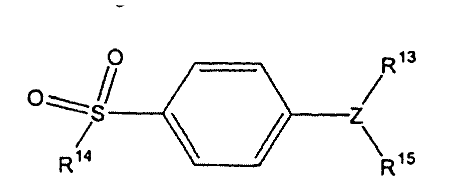 dans laquelle:
Z est choisi dans le groupe constitué par hétérocyclyle partiellement insaturé ou insaturé et des cycles carbocycliques partiellement insaturés ou insaturés;
R¹³ est choisi dans le groupe constitué par hétérocyclyle, cycloalkyle, cycloalcényle et aryle, R¹³ étant éventuellement substitué à des positions pouvant être substituées par un ou plusieurs radicaux choisis parmi alkyle, halogénoalkyle, cyano, carboxyle, alcoxycarbonyle, hydroxyle, hydroxyalkyle, halogénoalcoxy, amino, alkylamino, arylamino, nitro, alcoxyalkyle, alkylsulfinyle, halogéno, alcoxy et alkylthio;
R¹⁴ est choisi dans le groupe constitué par méthyle ou amino; et
R¹⁵ est choisi dans le groupe constitué par des radicaux choisis parmi H, halogéno, alkyle, alcényle, alcynyle, oxo, cyano, carboxyle, cyanoalkyle, hétérocyclyloxy, alkyloxy, alkylthio, alkylcarbonyle, cycloalkyle, aryle, halogénoalkyle, hétérocyclyle, cycloalcényle, aralkyle, hétérocyclylalkyle, acyle, alkylthioalkyle, hydroxyalkyle, alcoxycarbonyle, arylcarbonyle, aralkylcarbonyle, aralcényle, alcoxyalkyle, arylthioalkyle, aryloxyalkyle, aralkylthioalkyle, aralcoxyalkyle, alcoxyaralcoxyalkyle, alcoxycarbonylalkyle, aminocarbonyle, aminocarbonylalkyle, alkylaminocarbonyle, N-arylaminocarbonyle, N-alkyl-N-arylaminocarbonyle, alkylaminocarbonylalkyle, carboxyalkyle, alkylamino, N-arylamino, N-aralkylamino, N-alkyl-N-aralkylamino, N-alkyl-N-arylamino, aminoalkyle, alkylaminoalkyle, N-arylaminoalkyle, N-aralkylaminoalkyle, N-alkyl-N-aralkylaminoalkyle, N-alkyl-N-arylaminoalkyle, aryloxy, aralcoxy, arylthio, aralkylthio, alkylsulfinyle, alkylsulfonyle, aminosulfonyle, alkylaminosulfonyle, N-arylaminosulfonyle, arylsulfonyle, N-alkyl-N-arylaminosulfonyle;
dans la préparation d'un médicament destiné au traitement de la dépression, des épisodes dépressifs, des épisodes dépressifs récurrents, des épisodes maniaques et des troubles affectifs bipolaires.

2. Utilisation selon la revendication 1, **caractérisée en ce que** l'inhibiteur de COX-2 est choisi parmi le célécoxib, le rofécoxib, le déracoxib, le parécoxib, le valdécoxib, l'étoricoxib, l'ABT963 ou le JTE-522, leurs sels pharmaceutiquement acceptables ou leurs mélanges.

3. Utilisation selon la revendication. 2, **caractérisée en ce que** le célécoxib ou un de ses sels pharmaceutiquement acceptables est utilisé comme inhibiteur de COX-2.

4. Utilisation selon la revendication 3, **caractérisée en ce que** le célécoxib ou un de ses sels pharmaceutiquement acceptables doit être administré en une quantité de 50-1600 mg par jour, de préférence de 200 à 600 mg, au mieux de 400 mg.

5. Utilisation selon l'une quelconque des revendications précédentes, **caractérisée en ce que** le médicament doit être administré par voie orale.

6. Utilisation d'un inhibiteur de COX-2 tricyclique représenté par la structure générale de formule : 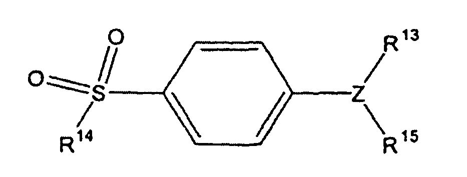 dans laquelle:
Z est choisi dans le groupe constitué par hétérocyclyle partiellement insaturé ou insaturé et des cycles carbocycliques partiellement insaturés ou insaturés;
R¹³ est choisi dans le groupe constitué par hétérocyclyle, cycloalkyle, cycloalcényle et aryle, R¹³ étant éventuellement substitué à des positions pouvant être substituées par un ou plusieurs radicaux choisis parmi alkyle, halogénoalkyle, cyano, carboxyle, alcoxycarbonyle, hydroxyle, hydroxyalkyle, halogénoalcoxy, amino, alkylamino, arylamino, nitro, alcoxyalkyle, alkylsulfinyle, halogéno, alcoxy et alkylthio;
R¹⁴ est choisi dans le groupe constitué par méthyle ou amino; et
R¹⁵ est choisi dans le groupe constitué par des radicaux choisis parmi H, halogéno, alkyle, alcényle, alcynyle, oxo, cyano, carboxyle, cyanoalkyle, hétérocyclyloxy, alkyloxy, alkylthio, alkylcarbonyle, cycloalkyle, aryle, halogénoalkyle, hétérocyclyle, cycloalcényle, aralkyle, hétérocyclylalkyle, acyle, alkylthioalkyle, hydroxyalkyle, alcoxycarbonyle, arylcarbonyle, aralkylcarbonyle, aralcényle, alcoxyalkyle, arylthioalkyle, aryloxyalkyle, aralkylthioalkyle, aralcoxyalkyle, alcoxyaralcoxyalkyle, alcoxycarbonylalkyle, aminocarbonyle, aminocarbonylalkyle, alkylaminocarbonyle, N-arylaminocarbonyle, N-alkyl-N-arylaminocarbonyle, alkylaminocarbonylalkyle, carboxyalkyle, alkylamino, N-arylamino, N-aralkylamino, N-alkyl-N-aralkylamino, N-alkyl-N-arylamino, aminoalkyle, alkylaminoalkyle, N-arylaminoalkyle, N-aralkylaminoalkyle, N-alkyl-N-aralkylaminoalkyle, N-alkyl-N-arylaminoalkyle, aryloxy, aralcoxy, arylthio, aralkylthio, alkylsulfinyle, alkylsulfonyle, aminosulfonyle, alkylaminosulfonyle, N-arylaminosulfonyle, arylsulfonyle, N-alkyl-N-arylaminosulfonyle;
en combinaison avec un médicament neuroleptique ou un antidépresseur dans la préparation d'un médicament ou de médicaments destinés au traitement des épisodes dépressifs, des épisodes dépressifs récurrents, des épisodes maniaques et des troubles effectifs bipolaires.

7. Utilisation selon la revendication 6, **caractérisée en ce que** l'inhibiteur de COX-2 est choisi parmi le célécoxib, le rofécoxib, le déracoxib, le parécoxib, le valdécoxib, l'étoricoxib, l'ABT963 ou le JTE-522, leurs sels pharmaceutiquement acceptables ou leurs mélanges.

8. Utilisation selon la revendication 7, **caractérisée en ce que** le célécoxib ou un de ses sels pharmaceutiquement acceptables est utilisé comme inhibiteur de COX-2.

9. Utilisation selon l'une quelconque des revendications 6 à 8, **caractérisé en ce que** le neuroleptique est choisi parmi la clozapine, l'olanzapine, la ziprasidone, la rispéridone, l'aripiprazole, la quétiapine, le fumarate de quétiapine, le sertindole, l'amisulpride, l'halopéridol, le décanoate d'halopéridol, le lactate d'halopéridol, la chlorpromazine, la fluphénazine, le décanoate de fluphénazine, l'énanthate de fluphénazine, le chlorhydrate de fluphénazine, le thiothixène, le chlorhydrate de thiothixène, la trifluopérazine, la perphénazine, l'amitriptyline, la thioridazine, la mésoridazine, la molindone, le chlorhydrate de molindone, la loxapine, le chlorhydrate de loxapine, le succinate de loxapine, le pimozide, le flupenthixol, la promazine, la triflupromazine, le chlorprothixène, le dropéridol, l'actophénazine, la prochlorpérazine, la méthotriméprazine, la pipotiazine, la ziprasidone, l'hopéridone, le zuclopenthixol, et leurs mélanges.

10. Utilisation selon l'une quelconque des revendications 6 à 8, **caractérisé en ce que** l'antidépresseur est choisi parmi l'amitriptyline, l'oxyde d'amitriptyline, la désipramine, la dibenzépine, la dosulépine, la doxépine, la chlorimipramine, l'imipramine, la nortriptyline, la mansérine, la maprotiline, la trimipramine, la viloxazine, la trazodone, la néfazodone, la mirtazapine, la venlafaxine, la réboxédine, la tranylcypromine, la brofaromine, le moclobémide, le citalopram, la paroxétine, la fluoxétine, la fluvoxamine, la sertaline, le millepertuis (herbe de Saint-Jean) et leurs mélanges.

11. Utilisation selon la revendication 9, **caractérisée en ce que** la rispéridone ou l'aripiprazole est utilisé comme neuroleptique.

12. Utilisation selon la revendication 11, **caractérisée en ce que** le célécoxib ou un de ses sels pharmaceutiquement acceptables et la rispéridone doivent être administrés en des quantités respectives de 50-1600 mg, de préférence de 200-600 mg, et de 2-6 mg.

13. Utilisation selon la revendication 12, **caractérisée en ce que** le célécoxib ou un de ses sels pharmaceutiquement acceptables et la rispéridone doivent être administrés en des quantités respectives de 400 mg et de 4-5 mg.

14. Utilisation selon la revendication 10, **caractérisé en ce que** la réboxétine est utilisée comme antidépresseur.

15. Utilisation selon l'une quelconque des revendications 6 à 14, **caractérisée en ce que** le médicament doit être administré par voie orale.

16. Utilisation selon l'une quelconque des revendications précédentes, **caractérisée en ce que** l'on doit administrer en outre un tranquillisant, de préférence le lorazépam.

17. Trousse d'éléments appropriée à l'utilisation dans le traitement de la dépression, des épisodes dépressifs, des épisodes dépressifs récurrents, des épisodes maniaques et des troubles effectifs bipolaires, comprenant une première forme galénique comprenant un médicament neuroleptique ou un antidépresseur et une deuxième forme galénique comprenant un inhibiteur de COX-2 tricyclique représenté par la structure générale de formule : 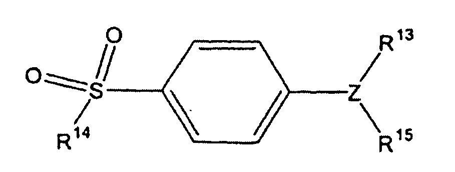 dans laquelle:
Z est choisi dans le groupe constitué par hétérocyclyle partiellement insaturé ou insaturé et des cycles carbocycliques partiellement insaturés ou insaturés;
R¹³ est choisi dans le groupe constitué par hétérocyclyle, cycloalkyle, cycloalcényle et aryle, R¹³ étant éventuellement substitué à des positions pouvant être substituées par un ou plusieurs radicaux choisis parmi alkyle, halogénoalkyle, cyano, carboxyle, alcoxycarbonyle, hydroxyle, hydroxyalkyle, halogénoalcoxy, amino, alkylamino, arylamino, nitro, alcoxyalkyle, alkylsulfinyle, halogéno, alcoxy et alkylthio;
R¹⁴ est choisi dans le groupe constitué par méthyle ou amino; et
R¹⁵ est choisi dans le groupe constitué par des radicaux choisis parmi H, halogéno, alkyle, alcényle, alcynyle, oxo, cyano, carboxyle, cyanoalkyle, hétérocyclyloxy, alkyloxy, alkylthio, alkylcarbonyle, cycloalkyle, aryle, halogénoalkyle, hétérocyclyle, cycloalcényle, aralkyle, hétérocyclylalkyle, acyle, alkylthioalkyle, hydroxyalkyle, alcoxycarbonyle, arylcarbonyle, aralkylcarbonyle, aralcényle, alcoxyalkyle, arylthioalkyle, aryloxyalkyle, aralkylthioalkyle, aralcoxyalkyle, alcoxyaralcoxyalkyle, alcoxycarbonylalkyle, aminocarbonyle, aminocarbonylalkyle, alkylaminocarbonyle, N-arylaminocarbonyle, N-alkyl-N-arylaminocarbonyle, alkylaminocarbonylalkyle, carboxyalkyle, alkylamino, N-arylamino, N-aralkylamino, N-alkyl-N-aralkylamino, N-alkyl-N-arylamino, aminoalkyle, alkylaminoalkyle, N-arylaminoalkyle, N-aralkylaminoalkyle, N-alkyl-N-aralkylaminoalkyle, N-alkyl-N-arylaminoalkyle, aryloxy, aralcoxy, arylthio, aralkylthio, alkylsulfinyle, alkylsulfonyle, aminosulfonyle, alkylaminosulfonyle, N-arylaminosulfonyle, arylsulfonyle, N-alkyl-N-arylaminosulfonyle;
pour une administration simultanée, séparée ou séquentielle.

18. Trousse d'éléments selon la revendication 17, **caractérisée en ce que** le neuroleptique est choisi parmi la clozapine, l'olanzapine, la ziprasidone, la rispéridone, la quétiapine, le fumarate de quétiapine, le sertindole, l'amisulpride, l'halopéridol, le décanoate d'halopéridol, le lactate d'halopéridol, la chlorpromazine, la fluphénazine, le décanoate de fluphénazine, l'énanthate de fluphénazine, le chlorhydrate de fluphénazine, le thiothixène, le chlorhydrate de thiothixène, la trifluopérazine, la perphénazine, l'amitriptyline, la thioridazine, la mésoridazine, la molindone, le chlorhydrate de molindone, la loxapine, le chlorhydrate de loxapine, le succinate de loxapine, le pimozide, le flupenthixol, la promazine, la triflupromazine, le chlorprothixène, le dropéridol, l'actophénazine, la prochlorpérazine, la méthotriméprazine, la pipotiazine, la ziprasidone, l'hopéridone, le zuclopenthixol et leurs mélanges.

19. Trousse d'éléments selon la revendication 17, **caractérisé en ce que** l'antidépresseur est choisi parmi l'amitryyotyline, l'oxyde d'amitriptyline, la désipramine, la dibenzépine, la dosulépine, la doxépine, la chlorimipramine, l'imipramine, la nortriptyline, la mansérine, la maprotiline, la trimipramine, la viloxazine, la trazodone, la néfazodone, la mirtazapine, la venlafaxine, la réboxéhine, la tranylcypromine, la brofaromine, le moclobémide, le citalopram, la paroxétine, la fluoxétine, la sertaline, le millepertuis (herbe de Saint-Jean) et leurs mélanges.

20. Trousse d'éléments selon l'une quelconque des revendications 17 à 19, **caractérisée en ce que** l'inhibiteur de COX-2 est choisi parmi le célécoxib, le rofécoxib, le parécoxib, le valdécoxib, l'étoricoxib, l'ABT963 ou le JTE-552, leurs sels pharmaceutiquement acceptables ou leurs mélanges.

21. Trousse d'éléments selon la revendication 18, **caractérisée en ce qu'**elle comprend du célécoxib ou un de ses sels pharmaceutiquement acceptables et de la rispéridone comme médicament neuroleptique.

22. Trousse d'éléments selon la revendication 19, **caractérisée en ce qu'**elle comprend du célécoxib ou un de ses sels pharmaceutiquement acceptables et de la réboxétrine comme antidépresseur.
